# DEMANDE DE BREVET EUROPEEN

(11) **EP 1 014 707 A1**
(43) Date de publication de la demande: **28.06.2000**
(21) Numéro de dépôt: 99403055.9
(22) Date de dépôt: 07.12.1999
(51) Int. Cl.: H04N 5/232

(54) **Caméra numérique et procédé de commande numérique associé**

(30) Priorité: 15.12.1998 FR 9815832
(71) Demandeur: THOMSON-CSF, 75008 Paris (FR)
(72) Inventeur: Eouzan, Jean-Yves, 94117 Arcueil Cedex (FR); Barnaud, Christophe, 94117 Arcueil Cedex (FR)
(74) Mandataire: Albert, Claude

(57) **Abrégé**

L'invention concerne une caméra numérique de visualisation en environnement évolutif et un procédé de commande numérique associé. Le procédé s'applique plus particulièrement aux caméras numériques pour l'endoscopie. Il permet à un utilisateur d'accéder à un très grand nombre de paramètres de réglages tout en n'utilisant qu'un nombre limité de données d'entrées.

Selon un exemple, la caméra selon l'invention est constituée d'une tête de détection (TD) électro-optique et d'un coffret de traitement (CTC) relié à la tête de détection. Le coffret est muni d'un clavier (CFA), et est relié à une télécommande (TCD). Le coffret de traitement (CTC) contient des fonctions numériques pré-enregistrées permettant chacune le réglage d'au moins un paramètre de la caméra, ainsi que des options caractéristiques d'un environnement donné et regroupant un ensemble desdites fonctions. Il contient en outre, notamment
- un module de surveillance des données entrées par l'utilisateur, par exemple au moyen du clavier (CFA) ou de la télécommande, et représentant les fonctions et/ou options choisies;
- un module de traitement des données correspondant directement à des fonctions ou à des options choisies par l'utilisateur afin de procéder aux réglages de la caméra correspondants.

## Description

L'invention concerne une caméra numérique de visualisation en environnement évolutif et un procédé de commande numérique associé. Elle s'applique essentiellement aux caméras évoluant en milieu biomédical. Plus particulièrement, l'invention concerne la commande des caméras couleur endoscopiques utilisées en milieu hospitalier pour mener les opérations chirurgicales sous endoscopie.

Les caméras endoscopiques sont habituellement constituées d'une tête de détection électro-optique connectée à un endoscope et reliée à une unité de contrôle, l'endoscope étant introduit à l'intérieur d'un patient. Une source de lumière, également reliée à l'endoscope, fournit l'éclairement nécessaire pour observer la scène. La caméra fournit une vidéo couleur à un ou plusieurs moniteurs situés dans le bloc opératoire. Au moyen de boutons de réglage disposés sur la face avant de l'unité de contrôle et/ou se trouvant directement sur la tête de détection de la caméra, un médecin peut avoir accès aux réglages de quelques paramètres de la caméra; par exemple, le gain et la balance des blancs. Ces réglages sont faits de façon analogique et sont en nombre limité ce qui permet de s'adapter plus ou moins bien aux conditions dans lesquelles la caméra évolue.

Cependant, dans un milieu évolutif tel que celui du corps humain dans lequel il existe une multitude de situations interventionnelles (type de cavité, nature des tissus, milieu, etc.), ces réglages sont tout à fait insuffisants pour optimiser la qualité de l'image et travailler dans les meilleures conditions. Or il n'est pas possible de rendre accessible au médecin un plus grand nombre de paramètres. D'une part car celui-ci n'est pas a priori un spécialiste technique des caméras et ne maîtrise donc pas l'influence de chacun des paramètres. D'autre part, en jouant sur un grand nombre de paramètres, le risque est fort de finalement dérégler la caméra et de ne pouvoir revenir à la configuration souhaitée.

Pour pallier cet inconvénient, la présente invention propose de connecter la tête de la caméra à un coffret de traitement numérique, ce coffret commandant par exemple un ou plusieurs moniteurs et au moins un clavier (par exemple un clavier situé sur la face avant du coffret et une télécommande). Grâce à des fonctions pré-enregistrées dans le coffret de commande et permettant chacune le réglage numérique d'un nombre donné de paramètres de la caméra, le médecin peut, grâce au procédé selon l'invention, accéder à un très grand nombre de paramètres tout en n'utilisant qu'un nombre limité de données d'entrée.

Plus précisément, l'invention concerne un procédé de commande numérique d'une caméra de visualisation en environnement évolutif, la caméra comportant une pluralité de paramètres de réglage et étant constituée notamment d'une tête de détection (TD) électro-optique et d'un coffret de traitement (CTC) relié à la tête de détection, le coffret étant relié en outre à au moins un moniteur (VISU) de visualisation des images délivrées par la caméra, caractérisé en ce qu'il comprend:
- une phase préalable d'apprentissage permettant la programmation d'un nombre prédéterminé de fonctions de réglage numériques enregistrées dans le coffret de traitement et permettant chacune le réglage d'au moins un paramètre de la caméra, ainsi que d'un nombre prédéterminé d'options caractéristiques notamment d'un environnement donné, et regroupant un ensemble desdites fonctions, ces fonctions étant accessibles par un utilisateur, soit directement, soit sous la forme des options,
- lors de la mise sous tension du coffret de traitement par l'utilisateur,
   - une étape d'initialisation de la caméra permettant notamment le réglage des paramètres de la caméra dans une configuration standard,
   - la surveillance des données entrées par l'utilisateur, ces données représentant les fonctions et/ou les options choisies par l'utilisateur,
   - le traitement des données correspondant directement à des fonctions de réglage ou à des options choisies par l'utilisateur afin de procéder aux réglages de la caméra correspondants.

L'invention concerne également une caméra numérique mettant en oeuvre le procédé selon l'invention.

Avantageusement, le procédé selon l'invention comporte en outre une gestion de configuration personnalisée, permettant à l'utilisateur lors du choix d'une ou de plusieurs options formant une configuration personnalisée, d'enregistrer cette configuration. Ainsi, l'utilisateur peut, avec un minimum de commandes, obtenir des réglages adaptés à l'environnement dans lequel la caméra opère, changer très facilement de configuration et revenir si besoin est, à une configuration standard. Le procédé selon l'invention permet ainsi d'obtenir une qualité d'images nettement meilleure sans augmenter la complexité d'utilisation de la caméra.

D'autres caractéristiques et avantages de l'invention apparaîtront à la lecture de la description qui suit, illustrée par les figures annexées qui représentent:
- la figure 1, un exemple d'une caméra selon l'invention;
- la figure 2, une liste donnant des exemples de fonctions de réglage pré-enregistrées;
- la figure 3, une liste donnant des exemples d'options pré-enregistrées;
- la figure 4, les différentes étapes d'un exemple de mise en oeuvre du procédé selon l'invention.

Dans ces figures, les mêmes éléments sont indexés par des références identiques.

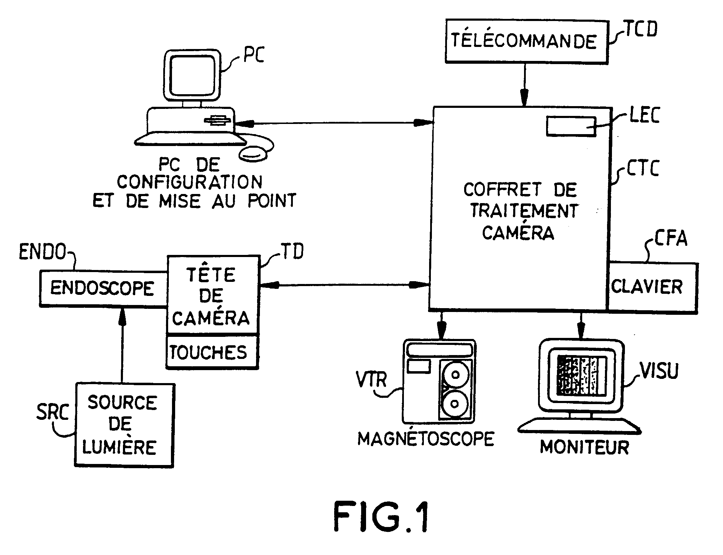

La figure 1 schématise un exemple de caméra numérique selon l'invention. Il s'agit d'une caméra endoscopique constituée d'une tête de détection TD, comportant par exemple trois détecteurs CCD (rouge, vert, bleu) et équipée de touches de réglage, connectée à un endoscope ENDO et reliée à un coffret de traitement CTC. Une source de lumière SRC, également reliée à l'endoscope, fournit l'éclairement nécessaire pour observer la scène. La caméra fournit une vidéo couleur à un ou plusieurs moniteurs VISU situés dans le bloc opératoire et permettant de visualiser les images délivrées par la caméra. Dans cet exemple, le coffret de traitement est équipé d'un clavier CFA positionné sur sa face avant et d'un lecteur LEC de cartes à puces. Une télécommande TCD munie de touches, est également reliée au coffret de traitement CTC. Un magnétoscope VTR est également prévu pour l'enregistrement d'images vidéo.

La caméra numérique comporte une pluralité de paramètres de réglage dont très peu étaient accessibles dans les caméras endoscopiques de l'art antérieur. En effet, il n'est pas possible de proposer tous ces réglages à l'utilisateur qui est, dans cet exemple, un membre du personnel hospitalier; celui-ci en effet n'est pas un spécialiste de la technique des caméras. Il risquerait de perdre un temps précieux et de dérégler la caméra sans pouvoir revenir à la configuration souhaitée.

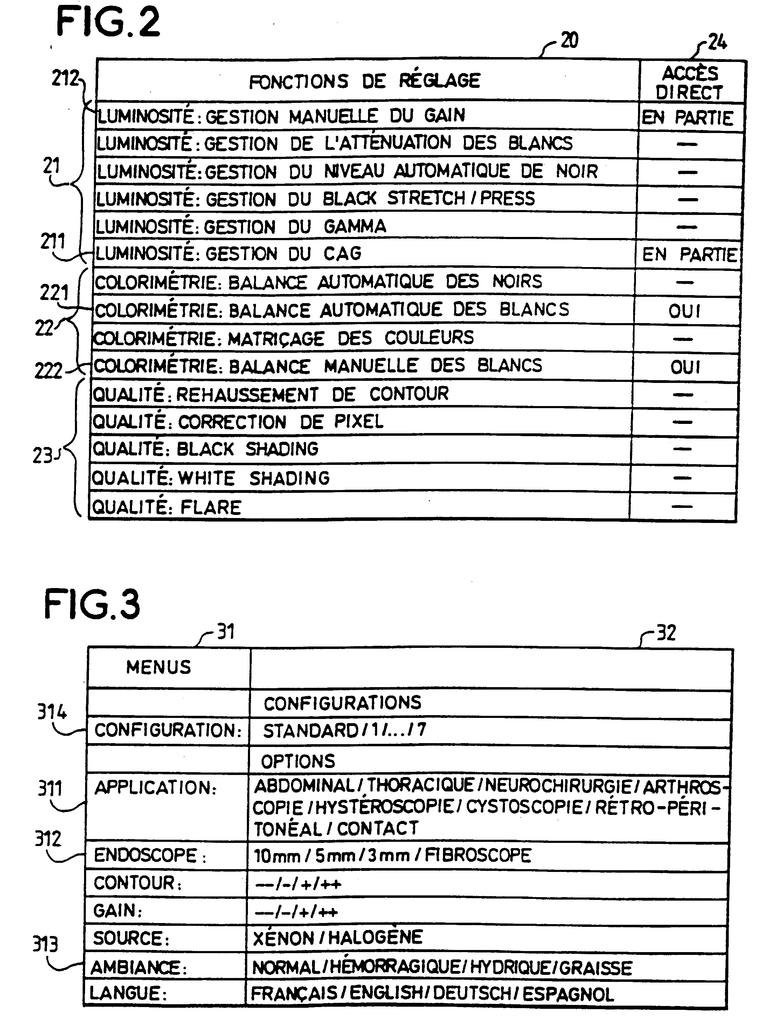

Dans la caméra selon l'invention, le coffret de traitement CTC contient notamment un nombre prédéterminé de fonctions numériques pré-enregistrées, permettant chacune le réglage d'au moins un paramètre de la caméra. Une liste non exhaustive indiquant des exemples de ces fonctions est donnée dans la colonne 20 du tableau de la figure 2. On trouve dans cet exemple des fonctions de réglage 21 relatives à la luminosité des images délivrées, des fonctions de réglage 22 relatives à la colorimétrie et des fonctions de réglage 23 relatives à la qualité des images. Certaines de ces fonctions font appel à plusieurs paramètres de réglage. Par exemple, la fonction 211 'Gestion du contrôle automatique de gain (CAG)' agit sur le CAG et le temps d'intégration des détecteurs CCD pour adapter le gain global de la caméra à la luminosité de la scène observée. Notons que si tous ces paramètres sont connus dans le domaine technique des caméras, ils sont pour la plupart inconnus des personnes qui travaillent avec les caméras endoscopiques car ils sont, dans les caméras de l'art antérieur, réglés en usine une fois pour toutes.

Dans la caméra selon l'invention, ces fonctions sont accessibles par l'utilisateur, soit directement, soit sous forme d'options regroupant un ensemble de ces fonctions. La colonne 24 du tableau de la figure 2 indique selon un exemple, lesquelles de ces fonctions sont accessibles directement. Il s'agit ici de la gestion manuelle du gain (212), de la gestion du contrôle automatique du gain (211), de la balance automatique des blancs (221), de la balance manuelle des blancs (222). Pour ces fonctions, des touches spécifiques sont prévues, par exemple sur le clavier face avant CFA du coffret CTC, ou sur la télécommande TCD, ou encore directement sur la tête de détection TD de la caméra. Pour la balance manuelle des blancs (222) par exemple, deux touches 'R' (Rouge) et'B' (bleu) accessibles sur le clavier de la face avant CFA ou sur la télécommande permettent d'égaliser les blancs, le niveau pouvant être indiqué au moyen d'un voyant lumineux sur une barre comprenant plusieurs niveaux. L'accessibilité à l'utilisateur de certaines fonctions peut également être limitée. Par exemple, la gestion manuelle du gain (212) peut être limitée à deux valeurs du gain (par exemple 0 et 6 dB) lorsque cette fonction est appelée directement par l'utilisateur.

Toutes les fonctions peuvent également être accessibles par l'utilisateur sous forme d'options regroupant un ensemble de ces fonctions. Ces options sont caractéristiques notamment d'un environnement donné et correspondent à des situations prédéterminées dans lesquelles peut évoluer la caméra. Elles permettent donc à un utilisateur non spécialiste de techniques video d'avoir accès à travers ces options à l'ensemble des paramètres de réglage correspondant à ces situations prédéterminées. Les options sont également pré-enregistrées dans le coffret de traitement CTC.

La figure 3 représente dans un exemple de caméra endoscopique selon l'invention, des options possibles pour l'utilisateur. Ces options sont par exemple réparties sous différents menus. Par exemple, le menu 'Application' (311) contient des options d'applications opérationnelles (par exemple 'abdominal', 'thoracique', 'neurochirurgie', etc. selon le type d'intervention effectuée). Le menu 'Endoscope' (312) contient différentes options sur le choix de l'endoscope ENDO utilisé. Le menu 'Ambiance' (313) recouvre des options significatives de l'environnement interventionnel (par exemple, 'normal', 'hémorragique', 'hydrique', 'graisse'). Chacune de ces options fait appel à une multitude de fonctions, qui, elles-mêmes, entraînent lorsqu'elles sont appelées les réglages des paramètres correspondants. Les options sont pré-enregistrées lors d'une phase préalable d'apprentissage au cours de tests réalisés dans les conditions des situations opérationnelles correspondant aux différentes options. Les options sont accessibles par l'utilisateur par exemple au moyen de quatre touches placées sur le clavier CFA de la face avant du coffret et/ou sur la télécommande. Par exemple, une première touche 'MENU' permet d'avoir accès aux différents menus. Une touche'VALID' permet de valider le choix qui est fait. Les deux autres touches symbolisant par exemple des flèches permettent d'avoir accès aux différentes options des menus. Ainsi, l'utilisateur a la possibilité à travers le choix d'options qui lui sont parlantes et qui s'appliquent directement à l'environnement opérationnel, d'avoir accès à l'ensemble des paramètres de réglage.

Le coffret de traitement de la caméra selon l'invention comprend en outre un module d'initialisation de la caméra permettant notamment le réglage des paramètres de la caméra dans une configuration standard. Lorsque l'utilisateur choisit une ou plusieurs options, cela définit une configuration personnalisée différente d'une configuration standard pré-enregistrée dans le coffret de traitement. L'utilisateur peut alors avoir la possibilité d'enregistrer cette configuration. Un menu 'configuration' accessible de la même façon que les autres, lui permet de choisir parmi l'ensemble des configurations, standard ou personnalisées.

Lorsque plusieurs utilisateurs sont amenés à utiliser la caméra selon l'invention, il est intéressant de prévoir une personnalisation de certaines fonctions de réglage, d'options ou de configurations utilisées spécifiquement par chacun, au moyen par exemple d'une carte à puce propre à chaque utilisateur. Cette carte peut être lue par le lecteur LEC, les données enregistrées étant gérées de la même façon que les autres, par le coffret de traitement.

Pour gérer les données entrées par l'utilisateur, le coffret de traitement CTC comprend un module de surveillance des données entrées par l'utilisateur, ces données représentant les fonctions et/ou les options choisies. Certaines données sont considérées de façon prioritaire à d'autres. Par exemple, les données entrées grâce aux touches de la tête de détection sont considérées de façon prioritaire. Entre les données entrées par le clavier CFA et les données entrées par les touches de la télécommande TCD, on pourra donner la priorité aux données entrées au moyen de la télécommande.

Le coffret de traitement de la caméra selon l'invention comprend également un module de traitement des données correspondant directement à des fonctions ou correspondant à des options choisies par l'utilisateur afin de procéder aux réglages de la caméra correspondants. Il peut comprendre en outre un module d'affichage permettant, pour chaque donnée entrée par l'utilisateur, une incrustation sur le moniteur correspondant à cette donnée afin de lui permettre de visualiser son choix. Cette possibilité est particulièrement intéressante lorsqu'un grand nombre d'options est présenté à l'utilisateur. En particulier, dans l'exemple précédent, le choix des options dans les menus peut se faire en visualisant les différents choix sur l'écran et en validant le choix grâce à la touche 'VALID'. Le coffret de traitement peut également comprendre une fonction pré-enregistrée supplémentaire, appelée ici 'STATUS', accessible par l'utilisateur au moyen d'une touche spécifique sur le clavier CFA, sur la télécommande ou sur la tête de détection, et permettant à tout moment d'afficher sur le moniteur VISU l'état des réglages ainsi que la configuration en cours.

La gestion numérique des fonctions de réglage se fait par exemple au moyen d'un micro-contrôleur mis en place dans le coffret de traitement CTC et commandant des circuits intégrés de type ASIC permettant le traitement sous forme numérique des signaux vidéo issus de la tête de détection de la caméra.

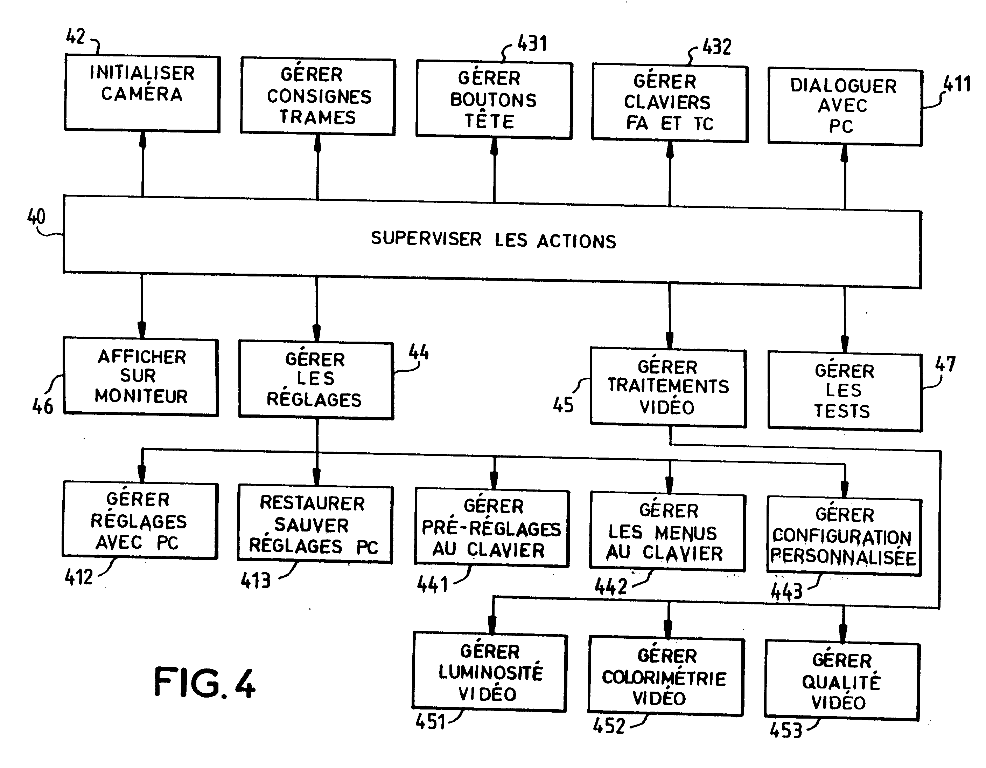

La figure 4 résume les différentes étapes du procédé selon l'invention dans un exemple particulier. Il est mis en oeuvre par exemple au moyen du micro-contrôleur qui permet de superviser l'ensemble des actions (étape 40).

Il comprend une phase préalable d'apprentissage permettant la programmation des fonctions de réglage et des options avant une utilisation opérationnelle de la caméra. Cette programmation peut se faire au moyen d'un ordinateur personnel (indiqué PC sur la figure 1, et noté PC dans la suite), permettant la configuration du micro-contrôleur et une éventuelle mise à jour des données. Dans l'exemple de la figure 4, cette phase d'apprentissage correspond aux étapes 411 de dialogue avec le PC, 412 de gestion des réglages avec le PC (permettant de tester des réglages en direct et préalablement à l'utilisation de la caméra par un utilisateur) et 413 de restauration/sauvegarde avec le PC. Cette dernière étape permet l'enregistrement des fonctions de réglage et des options.

Lors de la mise sous tension du coffret de traitement par l'utilisateur, le micro-contrôleur acquiert la configuration matérielle afin de déterminer le mode de fonctionnement en cours. Il détecte en particulier si le PC est connecté (il s'agit alors d'une phase d'apprentissage). En phase opérationnelle (PC non connecté), le procédé selon l'invention comprend une étape d'initialisation 42. Cette étape permet par exemple de détecter si la tête de détection est connectée. Lorsqu'elle est connectée, il est procédé à l'initialisation des traitement vidéo de la caméra et au réglage des paramètres de la caméra dans la configuration standard.

Le procédé de commande selon l'invention comprend en outre la surveillance des données entrées par l'utilisateur. Dans l'exemple de la figure 4, il s'agit de l'étape 431 de gestion des touches sur la tête de détection et de l'étape 432 de gestion du clavier CFA de la face avant et des touches de la télécommande. Il comprend d'autre part le traitement de ces données, qui correspondent comme cela a été expliqué ci-dessus, soit directement à des fonctions de réglage, soit à des options choisies par l'utilisateur. Le traitement des données comprend l'interprétation des données entrées (étape 'gérer les réglages' 44); l'étape 'gestion des pré-réglages' (441) correspond aux fonctions de réglages appelées directement, l'étape 'gérer les menus' (442) correspond aux options appelées grâce aux menus. Comme cela a été évoqué précédemment, une étape 'Gérer la configuration personnalisée' (443) permet avantageusement d'appeler une ou plusieurs options choisies par l'utilisateur et enregistrées sous forme d'une configuration. Le traitement des données comprend également la gestion des traitements vidéo (étape 45) correspondant aux fonctions appelées. Dans l'exemple évoqué précédemment, il s'agit de gérer la luminosité des images acquises (étape 451), la colorimétrie (étape 452), la qualité des images (étape 453).

Le procédé comporte également selon cet exemple, pour chaque donnée entrée par l'utilisateur, une incrustation sur le moniteur de visualisation correspondant à cette donnée et lui permettant de visualiser son choix (étape 45).

Le procédé de commande selon l'invention comprend en outre d'autres étapes utiles pour la qualité de la prise d'images et classiques des procédés de commande des caméras numériques. Par exemple, la 'gestion des tests' (étape 47) permet grâce à différents tests vidéo de valider le fonctionnement de la caméra.

## Revendications

1. Procédé de commande numérique d'une caméra de visualisation en environnement évolutif, la caméra comportant une pluralité de paramètres de réglage et étant constituée notamment d'une tête de détection (TD) électro-optique et d'un coffret de traitement (CTC) relié à la tête de détection, caractérisé en ce qu'il comprend:
- une phase préalable d'apprentissage permettant la programmation d'un nombre prédéterminé de fonctions de réglage numériques enregistrées dans le coffret de traitement et permettant chacune le réglage d'au moins un paramètre de la caméra, ainsi que d'un nombre prédéterminé d'options caractéristiques notamment d'un environnement donné, et regroupant un ensemble desdites fonctions, ces fonctions étant accessibles par un utilisateur, soit directement, soit sous la forme des options
- lors de la mise sous tension du coffret de traitement par l'utilisateur,
• une étape d'initialisation de la caméra permettant notamment le réglage des paramètres de la caméra dans une configuration standard,
• la surveillance des données entrées par l'utilisateur, ces données représentant les fonctions et/ou les options choisies par l'utilisateur,
• le traitement des données correspondant directement à des fonctions de réglage ou à des options choisies par l'utilisateur afin de procéder aux réglages de la caméra correspondants.

2. Procédé selon la revendication 1, caractérisé en ce que le coffret étant relié en outre à au moins un moniteur (VISU) de visualisation des images délivrées par la caméra, il comprend en outre pour chaque donnée entrée par l'utilisateur, une incrustation sur le moniteur correspondant à ladite donnée et lui permettant de visualiser son choix de configuration.

3. Procédé selon la revendication 2, caractérisé en ce qu'une fonction supplémentaire (STATUS) est accessible par l'utilisateur, cette fonction permettant notamment d'afficher sur au moins un moniteur de visualisation (VISU) l'état des réglages correspondant aux fonctions de réglage directement accessibles par l'utilisateur.

4. Procédé selon l'une des revendications précédentes, caractérisé en ce qu'il comprend en outre une gestion de configuration personnalisée, permettant à l'utilisateur lors du choix d'une ou de plusieurs options formant une configuration personnalisée, d'enregistrer cette configuration dans le coffret de traitement.

5. Procédé selon la revendication 1, caractérisé en ce que la tête de détection permettant l'acquisition d'images couleur, les fonctions de réglage permettent le réglage de paramètres liés à la luminosité, à la colorimétrie et à la qualité des images délivrées par la caméra.

6. Procédé de commande numérique d'une caméra endoscopique, la caméra comportant une pluralité de paramètres de réglage et étant constituée notamment d'une tête de détection (TD) électro-optique connectée à un endoscope (ENDO) pouvant être introduit à l'intérieur du corps du patient lors d'une intervention, et d'un coffret de traitement (CTC) relié à la tête de détection, caractérisé en ce qu'il comprend:
- une phase préalable d'apprentissage permettant la programmation d'un nombre prédéterminé de fonctions de réglage numériques enregistrées dans le coffret de traitement et permettant chacune le réglage d'au moins un paramètre de la caméra, ainsi que d'un nombre prédéterminé d'options caractéristiques notamment de situations interventionnelles, et regroupant un ensemble desdites fonctions, ces fonctions étant accessibles par un utilisateur, soit directement, soit sous la forme des options,
- lors de la mise sous tension du coffret de traitement par l'utilisateur,
• une étape d'initialisation de la caméra permettant notamment le réglage des paramètres de la caméra dans une configuration standard,
• la surveillance des données entrées par l'utilisateur, ces données représentant les fonctions et/ou les options choisies par l'utilisateur,
• le traitement des données correspondant directement à des fonctions de réglage ou à des options choisies par l'utilisateur afin de procéder aux réglages de la caméra correspondants.

7. Procédé selon la revendication 6, caractérisé en ce que lesdites options sont réparties en menus, ces menus pouvant être notamment l'application opérationnelle, le type d'endoscope (ENDO) utilisé, le choix de la source (SRC), l'environnement interventionnel.

8. Caméra numérique de visualisation en environnement évolutif, la caméra comportant une pluralité de réglages et étant constituée notamment d'une tête de détection (TD) électro-optique et d'un coffret de traitement (CTC) relié à la tête de détection, la caméra étant caractérisée en ce que le coffret de traitement contient notamment:
- un nombre prédéterminé de fonctions numériques pré-enregistrées et permettant chacune le réglage d'au moins un paramètre de la caméra, ainsi que d'un nombre prédéterminé d'options caractéristiques d'un environnement donné, et regroupant un ensemble desdites fonctions, ces fonctions étant accessibles par un utilisateur, soit directement, soit sous la forme des options,
- un module d'initialisation de la caméra permettant notamment le réglage des paramètres de la caméra dans une configuration standard,
- un module de surveillance des données entrées par l'utilisateur, ces données représentant les fonctions et/ou les options choisies par l'utilisateur,
- un module de traitement des données correspondant directement à des fonctions ou à des options choisies par l'utilisateur afin de procéder aux réglages de la caméra correspondants.

9. Caméra selon la revendication 8, caractérisée en ce que le coffret étant relié en outre à au moins un moniteur (VISU) de visualisation des images délivrées par la caméra, le coffret de traitement contient en outre un module d'affichage permettant, pour chaque donnée entrée par l'utilisateur, une incrustation sur le moniteur correspondant à ladite donnée et lui permettant de visualiser son choix de configuration.

10. Caméra selon l'une des revendications 8 ou 9, caractérisée en ce que les données sont entrées par l'utilisateur au moyen d'un clavier (CFA) positionné sur la face avant du coffret de traitement.

11. Caméra l'une des revendications 8 ou 9, caractérisée en ce que les données sont entrées par l'utilisateur au moyen de touches accessibles sur une télécommande (TCD).

12. Caméra l'une des revendications 8 ou 9, caractérisée en ce que les données sont entrées par l'utilisateur au moyen de touches accessibles sur la tête de détection (TD) de la caméra, les données entrées par ces boutons étant traitées de manière prioritaire.

13. Caméra l'une des revendications 8 ou 9, caractérisée en ce que le coffret de traitement (CTC) est équipé d'un lecteur (LEC) de cartes à puces, des données personnalisées pouvant être entrées par un utilisateur au moyen d'une carte à puce qui lui est propre, introduite dans le lecteur (LEC).

14. Caméra endoscopique, la caméra comportant une pluralité de réglages et étant constituée notamment d'une tête de détection (TD) électro-optique connectée à un endoscope (ENDO) pouvant être introduit à l'intérieur du corps d'un patient lors d'une intervention et d'un coffret de traitement (CTC) relié à la tête de détection, la caméra étant caractérisée en ce que le coffret de traitement contient notamment:
- un nombre prédéterminé de fonctions numériques pré-enregistrées et permettant chacune le réglage d'au moins un paramètre de la caméra, ainsi que d'un nombre prédéterminé d'options caractéristiques d'un environnement donné, et regroupant un ensemble desdites fonctions, ces fonctions étant accessibles par un utilisateur, soit directement, soit sous la forme des options,
- un module d'initialisation de la caméra permettant notamment le réglage des paramètres de la caméra dans une configuration standard,
- un module de surveillance des données entrées par l'utilisateur, ces données représentant les fonctions et/ou les options choisies par l'utilisateur,
- un module de traitement des données correspondant directement à des fonctions ou à des options choisies par l'utilisateur afin de procéder aux réglages de la caméra correspondants.

15. Caméra selon la revendication 14, caractérisée en ce que lesdites options sont réparties en menus, ces menus étant notamment l'application opérationnelle, le type d'endoscope (ENDO) utilisé, le choix de la source (SRC), l'environnement interventionnel.
